# EUROPEAN PATENT SPECIFICATION

(11) **EP 2 131 751 B1**
(45) Date of publication and mention of the grant of the patent: **25.05.2016**
(21) Application number: 08799753.2
(22) Date of filing: 04.02.2008
(51) Int. Cl.: A61B 17/16, A61B 17/88, A61B 17/34

(54) **POWERED DRIVER**
ELEKTRISCH BETRIEBENES ANTRIEBSGERÄT
COMMANDE MOTORISÉE

(30) Priority: 04.04.2007 US 910147 P
(43) Date of publication of application: 16.12.2009
(73) Proprietor: Vidacare LLC, Shavano Park, TX 78249-2095 (US)
(72) Inventor: MILLER, Larry, J., Spring Branch, Texas 78070 (US); TITKEMEYER, Robert, W., San Antonio, Texas 78216 (US); BOLLETER, David, S., San Antonio, Texas 78232 (US); TREVINO, Ruben, San Antonio, Texas 78258 (US); KILCOIN, Christopher, B., CA 95006-1977 (US); HARMON, Matthew, T., Santa Cruz, California 95062 (US)
(74) Representative: Lawrence, John
(86) International application number: PCT/US2008/052943
(87) International publication number: WO 2008/124206

(56) References cited:
- US-A- 3 022 596
- US-A- 3 734 207
- US-A- 4 940 459
- US-A- 6 110 174
- US-A1- 2003 114 858
- US-A1- 2003 205 987
- US-A1- 2003 225 411
- US-A1- 2004 064 136
- US-B2- 6 752 816

## Description

### STATEMENT OF GOVERNMENT INTEREST

Embodiments of present invention were developed using funds provided under United States Air Force contract number FA8650-06-C-6614. The U.S. government has certain rights in the invention.

### TECHNICAL FIELD

The present disclosure is related to apparatus for penetrating a bone and associated bone marrow with a powered driver and inserting an intraosseous device into the bone marrow.

### BACKGROUND OF THE DISCLOSURE

Every year, millions of patients are treated for life-threatening emergencies in the United States. Such emergencies include shock, trauma, cardiac arrest, drug overdoses, diabetic ketoacidosis, arrhythmias, burns, and status epilepticus just to name a few. For example, according to the American Heart Association, more than 1,500,000 patients suffer from heart attacks (myocardial infarctions) every year, with over 500,000 of them dying from its devastating complications.

Obtaining satisfactory vascular access may be a critical problem in approximately five (5%) percent to ten (10%) percent of patients treated in either prehospital or hospital settings. In the U.S. approximately six million patients annually may experience problems with traditional intravenous access. An essential element for treating medical emergencies is rapid establishment of vascular access to administer drugs and fluids directly into the circulatory system. Whether in an ambulance by paramedics, or in an emergency room by emergency specialists, the goal is the same - typically start an IV in order to administer life-saving drugs and fluids. To a large degree, the ability to successfully treat such critical emergencies is dependent on skill and luck of an operator in accomplishing vascular access.

While it is relatively easy to start an IV on many patients, doctors, nurses and paramedics often experience great difficulty establishing IV access in some patients. These patients are probed repeatedly with sharp needles in an attempt to solve this problem and may require an invasive procedure to finally establish an intravenous route. A further complicating factor in achieving IV access occurs "in the field" e.g. at the scene of an accident or during ambulance transport where it is difficult to see the target and excessive motion make accessing the venous system very difficult.

In the case of patients with chronic disease or the elderly, the availability of easily-accessible veins may be depleted. Other patients may have no available IV sites due to anatomical scarcity of peripheral veins, obesity, extreme dehydration or previous IV drug use. For these patients, finding a suitable site for administering lifesaving drugs becomes a monumental and frustrating task. While morbidity and mortality statistics are not generally available, it is known that many patients with life-threatening emergencies have died of ensuing complications because access to the vascular system with life-saving IV therapy was delayed or simply not possible. For such patients, an alternative approach is required.

Powered drivers associated with intraosseous (IO) devices typically include a housing with various types of motors and/or gear assemblies disposed therein. A rotatable shaft may be disposed within the housing and connected with a gear assembly. Various types of fittings, connections, connectors and/or connector receptacles may be provided at one end of the rotatable shaft extending from the housing to releasably engage an IO device with the powered driver.

US 2003/225411 discloses an apparatus and method for penetrating bone marrow. The apparatus includes a housing such as a handheld body, a penetrator assembly, a connector that releasably attaches the penetrator assembly to a drill shaft, a gear mechanism, a motor and a power supply and associated circuitry operable to power the motor. The penetrator assembly includes a removable inner trocar and an outer penetrator or needle. It also includes a grooved trocar that allows bone chips to be expelled as the apparatus is inserted into bone marrow.

US 3734207 discloses a surgical electric power drill with self-contained batteries suspended from the butt end of pistol grip handle in such an arrangement as to counterbalance the drill against top heaviness and toppling moments by locating the center of mass of the drill assembly within the pistol grip handle.

### SUMMARY OF THE DISCLOSURE

In accordance with teachings of the present disclosure, powered drivers are provided for use in gaining rapid access to a patient's vascular system.

The invention relates to an apparatus operable to insert an intraosseous device into a patient's bone marrow according to claim 1. Further embodiments of the invention are described in the dependent claims.

Powered drivers incorporating various teachings of the present disclosure may be used to provide intraosseous access to a patient's vascular system in the sternum, the proximal humerus (the shoulder area), the proximal tibia (below the knee) and the distal tibia (above the inside of the ankle).

### BRIEF DESCRIPTION OF THE DRAWINGS

A more complete and thorough understanding of the present embodiments and advantages thereof may be acquired by referring to the following description taken in conjunction with the accompanying drawings, in which like reference numbers indicate like features, and wherein:
FIGURE 1A is a schematic drawing showing an isometric view of one embodiment of a powered driver incorporating teachings of the present disclosure;
FIGURE 1B is a schematic drawing showing a distal end view of the powered driver of FIGURE 1A;
FIGURE 1C is a schematic drawing in section and in elevation taken along lines 1C-1C OF FIGURE 1B showing one example of a power supply incorporating teachings of the present disclosure.
FIGURE 1D is a schematic drawing in section showing one example of a powered drive having sound dampening materials in accordance with teachings of the present disclosure;
FIGURE 1E is a schematic drawing taken along lines 1E-1E of FIGURE 1B showing one example of a four battery power supply having a generally diamond shaped cross section;
FIGURE 2A is a schematic drawing showing an isometric view of a powered drive including a trigger guard and a lanyard attached thereto in accordance with teachings of the present disclosure;
FIGURE 2B is a schematic drawing in section and in elevation showing a powered driver having a power source, a trigger, electrical circuits, a motor and a gear assembly incorporating various teachings of the present disclosure;
FIGURE 2C is a schematic drawing showing a trigger guard in accordance with teachings of the present disclosure;
FIGURE 3 is a schematic drawing in section and in elevation with portions broken away showing another example of a powered drive having a housing, trigger assembly, motor, reduction gear and power source incorporating teachings of the present disclosure;
FIGURE 4A is a schematic drawing showing one example of an intraosseous needle set or penetrator assembly which may be inserted into a patient's vascular system using a powered driver incorporating teachings of the present disclosure;
FIGURE 4B is a schematic drawing showing an isometric view with portions broken away of a connector receptacle which may be releasably engaged with a powered driver incorporating teachings of the present disclosure;
FIGURE 5A is a schematic drawing in section with portions broken away showing one example of a protective cover in a first position blocking access to a trigger of a powered driver incorporating teachings of the present disclosure;
FIGURE 5B is a schematic drawing showing the protective cover of FIGURE 5A in a second position allowing access to the trigger of the powered driver;
FIGURE 5C is an isometric drawing showing another example of a trigger guard in a first position blocking access to a trigger of a powered driver incorporating teachings of the present disclosure; and
FIGURE 5D is a schematic drawing showing the trigger guard of FIGURE 5C in a second position allowing access to the trigger of the powered driver.

### DETAILED DESCRIPTION OF THE DISCLOSURE

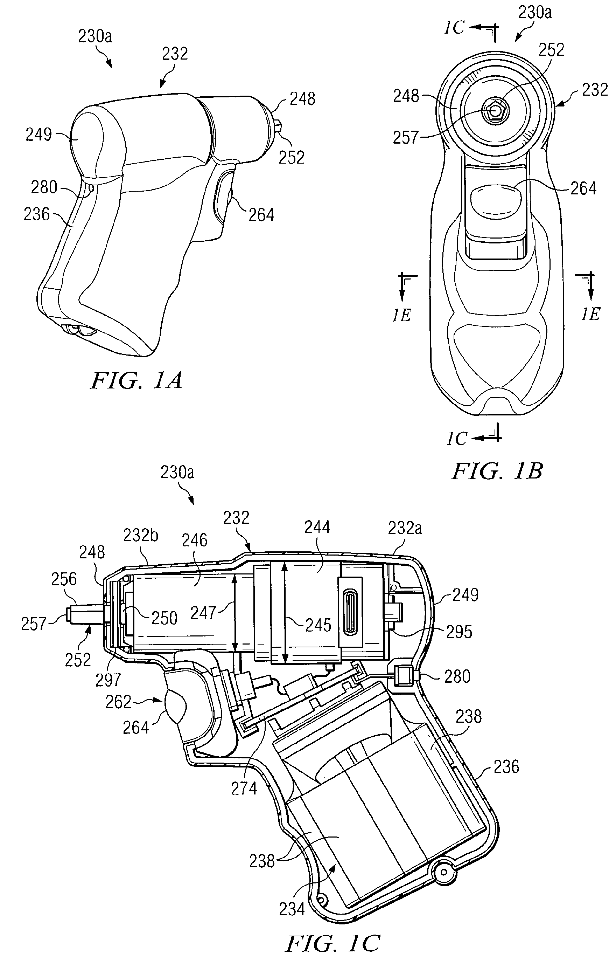

Preferred embodiments of the disclosure and its advantages may be best understood by reference to FIGURES 1A-5D wherein like numbers refer to same and like parts.

Vascular system access may be essential for treatment of many serious diseases, chronic conditions and acute emergency situations. Yet, many patients experience extreme difficulty obtaining effective treatment because of inability to obtain or maintain intravenous (IV) access. An intraosseous (IO) space provides a direct conduit to a patent's vascular system and systemic circulation. Therefore, IO access is generally an effective route to administer a wide variety of drugs, other medications and fluids equivalent to IV access. Rapid IO access or emergency vascular access (EVA) offers great promise for almost any serious emergency that requires vascular access to administer life saving drugs, other medications and/or fluids when traditional IV access is difficult or impossible.

Bone marrow typically includes blood, blood forming cells, and connective tissue disposed in an intraosseous space or cavity surrounded by compact bone. Long bones such as the tibia typically have an elongated central cavity filled with yellow bone marrow and adipose or connective tissue. Such cavities may also be referred to as a "medullary cavity", "bone marrow cavity" and/or "intraosseous space."

Compact bone disposed near an anterior or dorsal surface may be referred to as "anterior compact bone" or "anterior bone cortex." Compact bone disposed farther from the dorsal or anterior surface may be referred to as "posterior compact bone" or "posterior bone cortex."

The upper tibia proximate a patient's knee or the humeral head proximate a patient's shoulder may be used as insertion sites for an IO device to establish access with the patient's vascular system. Sternal access may also be used as an insertion site. Availability of multiple intraosseous insertion sites and associated target areas in adjacent bone marrow have proven to be especially important in applications such as emergency treatment of battlefield casualties or other mass casualty situations. Teachings of the present disclosure may be used to obtain intraosseous access at a wide variety of insertion sites and target areas.

IO access may be used as a "bridge" for temporary fluid and/or drug therapy during emergency conditions until conventional IV sites can be found and used. Conventional IV sites often become available because fluids and/or medication provided via IO access may stabilize a patient and expand veins and other portions of a patient's vascular system. IO devices and associated procedures incorporating teachings of the present disclosure may become standard care for administering medications and fluids in situations when IV access is difficult or not possible.

Intraosseous access may be used as a "routine" procedure with chronic conditions which substantially reduce or eliminate availability of conventional IV sites. Examples of such chronic conditions may include, but are not limited to, dialysis patients, patients in intensive care units and epilepsy patients. Intraosseous devices and associated apparatus incorporating teachings of the present disclosure may be quickly and safely used to provide IO access to a patient's vascular system in difficult cases such as status epilepticus to give medical personnel an opportunity to administer crucial medications and/or fluids. Further examples of such acute and chronic conditions are listed near the end of this written description.

Apparatus and methods incorporating teachings of the present disclosure may include using a first IO needle set having a fifteen (15) gage cannula with a length of approximately fifteen (15) millimeters to establish vascular access for patients weighing between approximately three (3) kilograms and thirty nine (39) kilograms. A second IO needle set having a fifteen (15) gage cannula with an approximate length of twenty-five (25) millimeters may be used to establish vascular access for patients weighing forty (40) kilograms and greater.

The term "driver" may be used in this application to include any type of powered driver satisfactory for inserting an intraosseous (IO) device such as a penetrator assembly, a catheter, an IO needle or an IO needle set into a selected portion of a patient's vascular system. Various techniques may be satisfactorily used to releasably engage or attach an IO device with a driver incorporating teachings of the present disclosure. A wide variety of connectors and associated connector receptacles, fittings and/or other types of connections with various dimensions and configurations may be satisfactorily used to releasably engage an IO device with a driver. A battery powered driver incorporating teachings of the present disclosure may be used to insert an intraosseous device into a selected target area in ten seconds or less. The reduced size and weight of drivers incorporating teachings of the present disclosure may accommodate use in emergency medical vehicles and emergency crash carts at medical facilities and/or carrying in backpacks of military personnel deployed for extended periods of time in remote locations.

The term "intraosseous (IO) device" may be used in this application to include any hollow needle, hollow drill bit, penetrator assembly, bone penetrator, catheter, cannula, trocar, inner penetrator, outer penetrator, IO needle or IO needle set operable to provide access to an intraosseous space or interior portions of a bone.

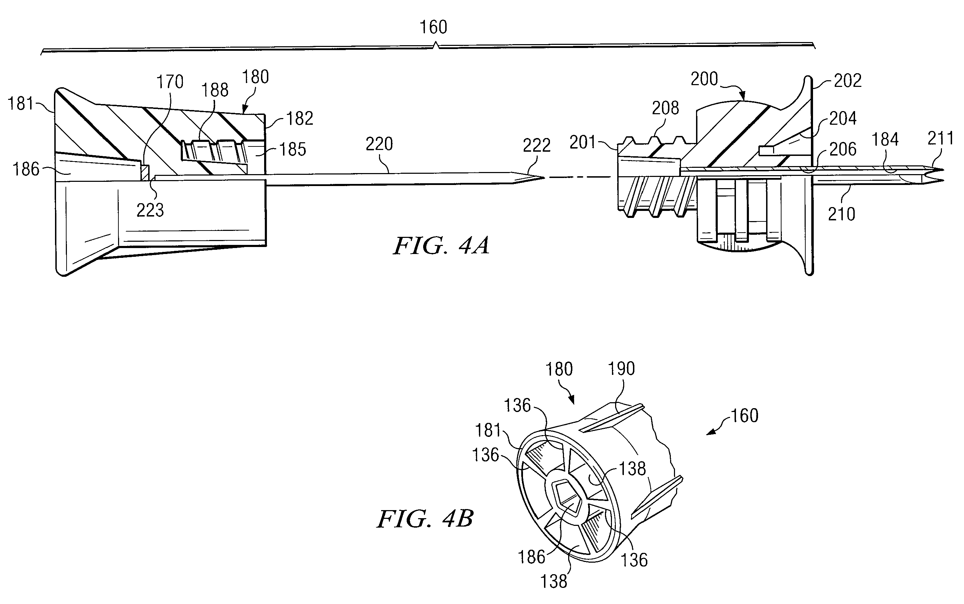

For some applications an IO needle or IO needle set may include a connector with a trocar or stylet extending from a first one end of the connector. A second end of the connector may be operable to be releasably engaged with a powered driver incorporating teachings of the present disclosure. An IO needle or IO needle set may also include a hub with a hollow cannula or catheter extending from a first end of the hub. A second end of the hub may include an opening sized to allow inserting the trocar through the opening and the attached hollow cannula. The second end of the hub may be operable to be releasably engaged with the first end of the connector. As previously noted, the second end of the connector may be releasably engaged with a powered driver. A wide variety of connectors and hubs may be used with an IO device incorporating teaching of the present disclosure. The present disclosure is not limited to connector 180 or hub 200 as shown in FIGURES 4A and 4B.

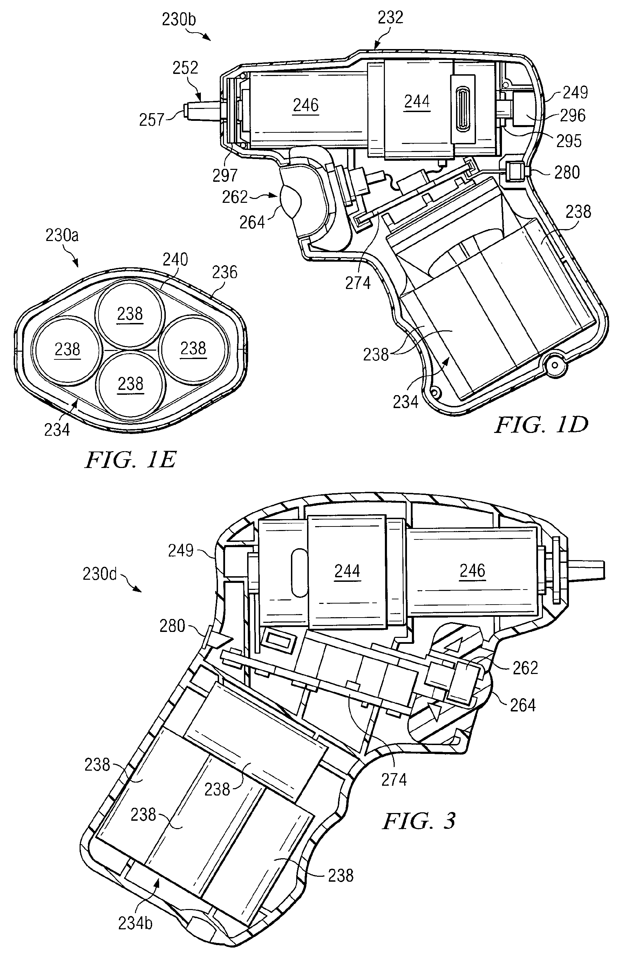

Various features of the present disclosure may be described with respect to powered drivers 230a-230d, 330a and 330b (See FIGURE 3). Various features of the present disclosure may also be described with respect to intraosseous device 160 such as shown in FIGURES 4A and 4B. However, the present disclosure is not limited to use with intraosseous device 160 or powered drivers 230a-230d, 330a and 330b.

Powered driver 230a as shown in FIGURES 1A, 1B, 1C, and 1E may be satisfactorily used to insert an intraosseous device at a desired insertion site adjacent to a bone and associated bone marrow. Powered driver 230a as shown in FIGURES 1A, 1B, 1C, and 1E may include one or more features of the present disclosure. At least one or more features of the present disclosure may also be shown with respect to powered drivers 230b, 230c, 230d, 330a, and 330b (See FIGURE 3).

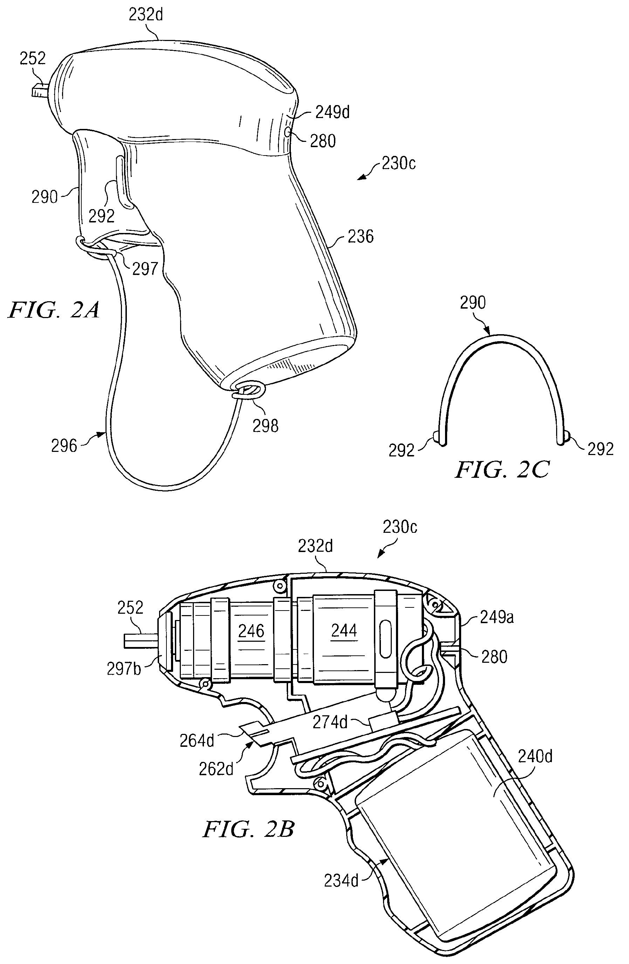

Powered driver 230a may include housing 232 having the general configuration of a small pistol defined in part by handle 236. Various components associated with powered driver 230a may be disposed within housing 232 including handle 236. For example a power source such as battery pack 234 may be disposed within handle 236. Battery pack 234 may have various configurations and may include multiple batteries 238 disposed within sealed packaging material 240 (See FIGURE 1E). For purposes of describing various features of the present disclosure sealed packaging material 240 is not shown in FIGURES 1C, 1D and 2A.

Handle 236 may be generally described as an elongated, hollow container sized to receive battery pack or power supply 234 therein. Housing 230a including handle 236 may be formed from relatively strong, heavy duty polymeric material. For some applications housing 232 may be formed in two halves which are joined together to form a fluid tight seal with the various components associated with powered driver 230a disposed therein. For such applications power source or power supply 234 may include disposable batteries. In other embodiments, handle 236 may include exterior connections configured to allow recharging of power supply 234.

Motor 244 and gear assembly 246 may be disposed within portions of housing 232 adjacent to handle 236. For embodiments represented by powered drivers 230a-e, respective motor 244 and gear assembly 246 may be generally aligned with each other. Motor 244 may be rotatably engaged with one end of gear assembly 246. Drive shaft 250 may be rotatably engaged with and extend from another end of gear assembly 246 opposite from motor 244.

Distal end or first end 248 of housing 232 may include opening (not expressly shown) with portions of drive shaft 250 extending therefrom. For some applications end 252 of drive shaft 250 extending from housing 232 may have a generally pentagonal shaped cross section with tapered surfaces 256 disposed thereon. Tapered surfaces 256 may be disposed at an angle of approximately three (3°) degrees with respect to a longitudinal axis or rotational axis (not expressly shown) associated with drive shaft 250. Relatively small magnet 257 disposed on the extreme end of drive shaft 250 opposite from distal end 248 of housing 232. Fittings and/or connectors with various dimensions and/or configurations other than end 252 of drive shaft 250 and/or magnet 257 may also be satisfactorily used with a powered driver incorporating teachings of the present disclosure.

Intraosseous devices having corresponding tapered openings or connector receptacles may be releasably engaged with end 252 of drive shaft 250 extending from housing 232. For example, end 252 extending from distal end 248 of housing 232 may be releasably engaged with tapered opening 186 in connector 180 as shown in FIGURES 4A and 4B. Tapered opening 186 and end 252 of drive shaft 250 preferably have non-sticking tapers.

For some applications both motor 244 and gear assembly 246 may have generally cylindrical configurations. Exterior portion 245 of motor 244 may correspond with the largest outside diameter or nominal outside diameter associated with motor 244. Exterior portion 247 of gear assembly 246 may correspond with the largest outside diameter or nominal outside diameter associated with gear assembly 246. For embodiments of the present disclosure represented by powered drivers 230a-230b, exterior portion 245 of motor 244 may have a nominal outside diameter portion larger than any outside diameter portion associated with gear assembly 246.

Portions of housing 232 may have generally similar cylindrical configurations corresponding with exterior portions of motor 244 and gear assembly 246. For example, segment 232a of housing 232 may have a generally cylindrical, hollow configuration with an inside diameter compatible with exterior portion 245 of motor 244. Housing segment 232b may have a generally cylindrical, hollow configuration with an inside diameter compatible with exterior portion 247 of gear assembly 246. Since portions of motor 244 have an outside diameter that is larger than the outside diameter of any portion of gear assembly 246, housing segment 32a may have a larger outside diameter than the outside diameter of housing segment 32b.

Motors and gear assemblies satisfactory for use with a powered driver incorporating teachings of the present disclosure may be obtained from various vendors. Such motor and gear assemblies are typically ordered as "sets" with one end of each motor securely attached to an adjacent end of an associated gear assembly. A drive shaft having various dimensions and/or configurations may extend from the gear assembly opposite from the motor. The gear assemblies may sometimes be referred to as "reduction gears" or "planetary gears". The dimensions and/or configurations of an associated housing may be modified to accommodate an associated motor and gear assembly.

For some applications a thrust pad structure 295 may be disposed between proximal end 249 of housing 232 and adjacent portions of motor 244. The thrust pad structure 297 may also be disposed adjacent to distal end 248 of housing 232. Thrust pad structures 295 and 297 may limit longitudinal movement of motor 244, gear assembly 246 and drive shaft 250 within associated portions of housing 232. Thrust pad structures 295 and 297 are also shown in FIGURES 1C and 1F. In some embodiments, thrust pad structure 295 may include one or more fixed portions of motor 244 configured to locate motor 244 in relation to housing 232.

For embodiments represented by power driver 230b as shown in FIGURE 1D sound damping material 296 may be disposed adjacent to proximal end 249 of housing 232. One of the features of the present invention may include placing sound damping material at various locations within a housing to reduce noise associated with operating a powered driver while inserting an intraosseous device into a target area.

For embodiments of the present disclosure such as shown in FIGURE 1D powered driver 230b may include electronic circuit board or smart board 274. Electrical circuit board 274 may also provide an electronic safety switch operable to turn off or deenergize motor 244 after power driver 230b has been energized for a selected amount of time. For example, electronic circuit board 274 may turn off power driver 230b after approximately ten seconds. For other applications, electronic circuit board 274 may turn off motor 244 after 20 seconds of operations. The time period for deenergizing motor 244 may be selected to save energy in an associated power source.

Powered drivers incorporating teaching of the present disclosure may typically run only a few seconds during insertion of an intraosseous device into bone and associated bone marrow. Activation of an associated motor for a longer period of time may imply a malfunction or use of the powered driver for other than normal IO insertion. To prevent draining of batteries, the timeout or timing circuit may automatically turn off the motor after a set time period such as ten (10) seconds or twenty (20) seconds. The time period may correspond with the length of time required to charge the capacitor shown in the above drawing. The timeout or timing circuit may function as a battery saving device. The timeout or timing circuit may be particularly important when a powered driver is carried in a tool box or a backpack to prevent accident draining of the battery.

Additional embodiments of the present disclosure are shown in FIGURES 2A, 2B, and 2C. For such embodiments trigger guard 290 may be releasably disposed over portions of trigger assembly 262d extending from housing 232d. Trigger guard 290 as shown in FIGURE 2A may be formed from relatively strong but flexible material. Trigger guard 290 may have a generally U shaped configuration with ribs 292 formed on opposite sides of trigger guard 290. When trigger guard 290 is disposed over end or button 264d of trigger assembly 292d, ribs 292 will preferably be disposed immediately adjacent to portions of handle 236. A pair of ribs 292 disposed on opposite sides of trigger guard 290 may be used to easily release trigger guard 290 from covering trigger assembly 262d. By placing respective ribs 292 on opposite sides of trigger guard 290, either the thumb (not expressly shown) of an operator's left hand or right hand may be used to remove trigger guard 290.

Another aspect of the present disclosure may include attaching a trigger guard with a housing using a lanyard. For example, lanyard 296 as shown in FIGURE 2A may be formed from various types of flexible, light weight material. First end 297 of lanyard 296 may be securely engaged with an appropriately sized opening formed in trigger guard 290. Second end 298 of lanyard 296 may be securely engaged on the end of handle 236. As a result of using lanyard 296, an operator may quickly release trigger guard 290 from power driver 230c but will avoid losing trigger guard 290.

As shown in FIGURE 2A, power driver 230c may also include electrical circuit board 274d operable to activate light 280. Powered drive 230c as shown in FIGURES 1A-E, 2A-B, and 3 may include light 280 operable to indicate the status of power supply 240d disposed within handle 236. Light 280 may be disposed at proximal end or second end 249 of powered driver 230c. For some applications light 280 may be activated when button 264 of trigger assembly 262 is activated or depressed. A green light may be used to indicate that battery pack 234b has sufficient power to activate motor 244 and gear assembly 246. A red light may be visible at indicator 280 to indicate that battery power 234b may be failing. A wide variety of indicators including, but not limited to, light emitting diodes (LED's), liquid crystal displays (LCD's) and small more conventional light bulbs may be satisfactorily used with a powered driver according to teachings of the present disclosure. In some embodiments, indicators may be covered by a user's hand when in use to avoid visibility (e.g., in some military situations).

Additional embodiments of the present disclosure are shown in FIGURES 3. For such embodiments a power supply 234b may include five batteries 238. The embodiment of the present disclosure as represented by powered driver 230c may also include one or more of the electronic circuit boards.

Penetrator assembly 160 as shown in FIGURES 4A and 4B may include connector 180, and associated hub 200, outer penetrator 210 and inner penetrator 220. Penetrator assembly 160 may include an outer penetrator such as a cannula, hollow tube or hollow drill bit and an inner penetrator such as a stylet or trocar. Various types of stylets and/or trocars may be disposed within an outer penetrator. For some applications outer penetrator or cannula 210 may be described as a generally elongated tube sized to receive inner penetrator or stylet 220 therein. Portions of inner penetrator 220 may be disposed within longitudinal passageway 184 extending through outer penetrator 210. The outside diameter of inner penetrator 220 and the inside diameter of longitudinal passageway 184 may be selected such that inner penetrator 220 may be slidably disposed within outer penetrator 210.

Metallic disc 170 may be disposed within opening 186 for use in releasably attaching connector 180 with magnet 257 disposed on end 252 of drive shaft 252. End 223 of inner penetrator 220 may be spaced from metallic disc 170 with insulating or electrically nonconductive material disposed therebetween.

Tip 211 of outer penetrator 210 and/or tip 222 of inner penetrator 220 may be operable to penetrate bone and associated bone marrow. The configuration of tips 211 and/or 222 may be selected to penetrate a bone or other body cavities with minimal trauma. First end or tip 222 of inner penetrator 220 may be trapezoid shaped and may include one or more cutting surfaces. In one embodiment outer penetrator 210 and inner penetrator 220 may be ground together as one unit during an associated manufacturing process. Providing a matching fit allows respective tips 211 and 222 to act as a single drilling unit which facilitates insertion and minimizes damage as portions of penetrator assembly 160 are inserted into a bone and associated bone marrow. Outer penetrator 210 and/or inner penetrator 220 may be formed from stainless steel, titanium or other materials of suitable strength and durability to penetrate bone.

Hub 200 may be used to stabilize penetrator assembly 160 during insertion of an associated penetrator into a patient's skin, soft tissue and adjacent bone at a selected insertion site. First end 201 of hub 200 may be operable for releasable engagement or attachment with associated connector 180. Second end 202 of hub 200 may have a size and configuration compatible with an associated insertion site for outer penetrator 210. The combination of hub 200 with outer penetrator 210 may sometimes be referred to as a "penetrator set" or intraosseous needle.

Connector 180 and attached inner penetrator 220 may be releasably engaged with each other by Luer type fittings, threaded connections or other suitable fittings formed on first end 201 of hub 200. Outer penetrator 210 extends from second end 202 of hub 200.

For some applications connector 180 may be described as a generally cylindrical tube defined in part by first end 181 and second end 182. The exterior of connector 180 may include an enlarged tapered portion adjacent to end 181. A plurality of longitudinal ridges 190 may be formed on the exterior of connector 180 to allow an operator to grasp associated penetrator assembly 160 during attachment with a drive shaft. Longitudinal ridges 190 also allow connector 180 to be grasped for disengagement from hub 200 when outer penetrator 210 has been inserted into a bone and associated bone marrow.

Second end 182 of connector 180 may include opening 185 sized to receive first end 201 of hub 200 therein. Threads 188 may be formed in opening 185 adjacent to second end 182 of connector 180. Threaded fitting 188 may be used in releasably attaching connector 180 with threaded fitting 208 adjacent to first end 201 of hub 200.

First end 201 of hub 200 may include a threaded connector 208 or other suitable fittings formed on the exterior thereof. First end 201 may have a generally cylindrical pin type configuration compatible with releasably engaging second end or box end 182 of connector 180.

For some applications end 202 of hub 200 may have the general configuration of a flange. Angular slot or groove 204 sized to receive one end of protective cover or needle cap 234 may be formed in end 202. Slot or groove 204 may be used to releasable engage a needle cover (not expressly shown) with penetrator assembly 160.

For some applications a penetrator assembly may include only a single, hollow penetrator. For other applications a penetrator assembly may include an outer penetrator such as a cannula, hollow needle or hollow drill bit and an inner penetrator such as a stylet, trocar or other removable device disposed within the outer penetrator. Penetrator 210 is one example of a single, hollow penetrator or cannula.

The size of a penetrator may vary depending upon the intended application for the associated penetrator assembly. Penetrators may be relatively small for pediatric patients, medium size for adults and large for oversize adults. By way of example, a penetrator may range in length from five (5) mm to thirty (30) mm. The diameter of a penetrator may range from eighteen (18) gauge to ten (10) gauge. The length and diameter of the penetrator used in a particular application may depend on the size of a bone to which the apparatus may be applied. Penetrators may be provided in a wide variety of configurations depending upon intended clinical purposes for insertion of the associated penetrator. For example, there may be one configuration for administering drugs and/or fluids to a patient's bone marrow and an alternative configuration for sampling bone marrow and/or blood from a patient. Other configurations may be appropriate for bone and/or tissue biopsy.

For some applications connector 180 may be described as having a generally cylindrical configuration defined in part by first end 181 and second end 182. Exterior portions of connector 180 may include an enlarged tapered portion adjacent to end 181. A plurality of longitudinal ridges 190 may be formed on the exterior of connector 180 to allow an operator to grasp associated penetrator assembly 160 during attachment with a drive shaft. Longitudinal ridges 190 also allow connector 180 to be grasped for disengagement from hub 200 when outer penetrator 210 has been inserted into a bone and associated bone marrow.

First end 181 of connector of 180 may included opening 186 sized to receive portions drive shaft 52 therein. A plurality of webs 136 may extend radially outward from connector receptacle 186. Webs 136 cooperate with each other to form a plurality of openings 138 adjacent to first end 181. Opening 186 and openings 138 cooperate with each other to form portions of a connector receptacle operable to receive respective portions of a connector (not expressly shown) therein.

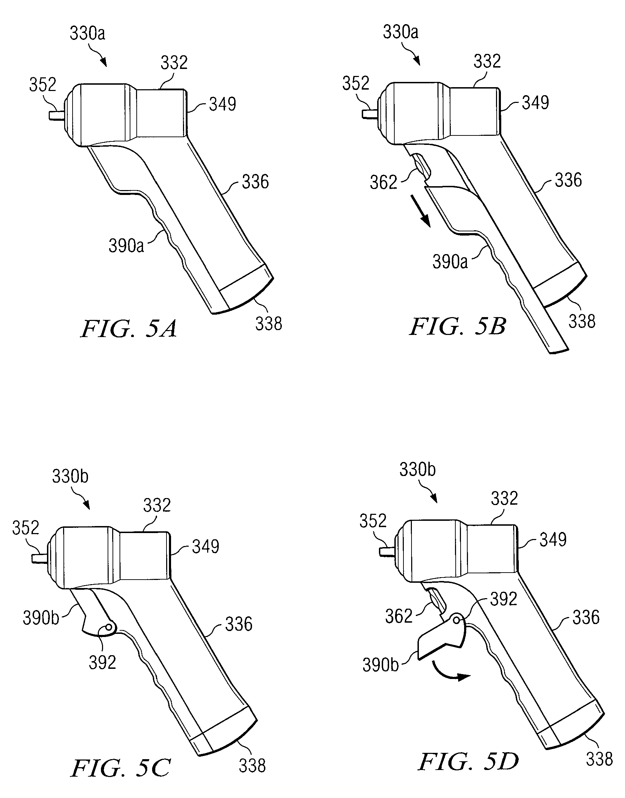

FIGURE 5A-5D show further examples of trigger guards which may be used with a powered driver in accordance with teachings of the present disclosure. Power drivers 330a and 330b may include cap 338 disposed on one end of handle 336. Cap 338 may be used to replace a power supply (not expressly shown) disposed within handle 336.

For some embodiments a trigger guard may be slidably disposed on portions of a handle adjacent to an associated trigger assembly. For example, FIGURE 5A shows power driver 330a with trigger guard 390a in its first position covering portions of trigger assembly 362. In FIGURE 5B, trigger guard 390a is shown in its second position which allows access to trigger assembly 362 for operation of power driver 330a. For such embodiments, trigger guard 390a may be described as having a generally elongated configuration with a U shaped cross section. The profile of the U shaped cross section of trigger guard 390a may be selected to correspond with adjacent portions of handle 336 and trigger assembly 362. The length of trigger guard 390a may correspond approximately to the length of adjacent portion of handle 336.

For embodiments represented by power driver 330b as shown in FIGURE 5C and 5D, trigger guard 390b may have a generally U shaped configuration similar to corresponding portions of trigger guard 390a. However, the length of trigger guard 390b may be substantially less than the length of trigger guard 390a. For embodiments such as shown in FIGURES 5C and 5D, pivot pin 392 may be disposed between portions of trigger guard 390b and adjacent portions of handle 396. Pivot pin 392 may allow rotational movement of trigger guard 390b from a first position blocking access to trigger assembly 362 as shown in FIGURE 5C and a second position allowing access to trigger assembly 362 as shown in FIGURE 5D.

Examples of acute and chronic conditions which may be treated using powered drivers, intraosseous devices, and procedures incorporating teachings of the present disclosure include, but are not limited to, the following:
**Anaphylaxis** (epinephrine, steroids, antihistamines, fluids, and life support)
**Arrhythmia** (anti-arrhythmics, electrolyte balance, life support);
**Burns** (fluid replacement, antibiotics, morphine for pain control);
**Cardiac arrest** (epinephrine, atropine, amiodarone, calcium, xylocaine, magnesium);
**Congestive heart failure** (life support, diuretics, morphine, nitroglycerin);
**Dehydration** (emergency port for life support, antibiotics, blood, electrolytes);
**Diabetic Ketoacidosis** (life support, electrolyte control, fluid replacement);
**Dialysis** (emergency port for life support, antibiotics, blood, electrolytes);
**Drug overdose** (naloxone, life support, electrolyte correction);
**Emphysema** (life support, beta adrenergics, steroids);
**Hemophiliacs** (life support, blood, fibrin products, analgesics);
**Osteomyelitis** (antibiotics directly into the site of infection, analgesics);
**Pediatric applications** (shock, dehydration, nutrition, electrolyte correction);
**Renal Failure** (both acute and chronic kidney failure, inability to purify blood);
**Seizures** (anti-seizure medications, life support, fluid balance);
**Shock** (life support fluids, pressor agents, antibiotics, steroids);
**Sickle cell crisis** (fluid, morphine for pain, blood, antibiotics); and
**Trauma** (emergency port for life support fluids, antibiotics, blood, electrolytes).

Although the present disclosure and its advantages have been described in detail, it should be understood that various changes, substitutions and alternations can be made herein without departing from the scope of the disclosure as defined by the following claims.

## Claims

1. An apparatus operable to insert an intraosseous device (160) into a bone and associated bone marrow comprising:
a housing (232) with a handle (236) extending therefrom and a drive shaft (250) extending from an opening in the housing, the drive shaft operable to releasably engage the intraosseous device, the handle configured as an elongated, hollow container sized to receive a power supply (234);
a motor (244) disposed within a first segment (232a) of the housing and rotatably engaged with the drive shaft;
a power supply (234) and associated electrical circuit (274) operable to power the motor, the power supply disposed within the handle (236);
a gear assembly (246) disposed between and rotatably engaged with the motor and the drive shaft, wherein the gear assembly is disposed within a second segment (232b) of the housing;
the motor and the gear assembly having respective generally cylindrical configurations;
the gear assembly having a nominal outside diameter portion smaller than a corresponding nominal outside diameter portion of the motor;
wherein the rotational axis of the drive shaft is aligned with the rotational axis of the motor; **characterized in that** the first segment (232a) of the housing has a larger nominal outside diameter than the second segment (232b) of the housing.

2. The apparatus of Claim 1 wherein the intraosseous device further comprises:
a penetrator assembly defined in part by a generally hollow, elongated cannula (210); and
a removable inner trocar (220) slidably disposed within the cannula.

3. The apparatus of Claim 1 further comprising:
a trigger assembly (262) having at least one portion extending from the housing; and
the trigger assembly operable to activate the motor to rotate the drive shaft.

4. The apparatus of Claim 1 wherein the power supply further comprises four batteries (238) disposed adjacent to each other in a generally diamond shaped pattern.

5. The apparatus of Claim 1 wherein the power supply further comprises:
four batteries disposed adjacent to each other in a generally diamond shaped pattern; and
a fifth battery disposed on one end of the four batteries.

6. The apparatus of Claim 1 wherein the power supply comprises:
a generally diamond shaped cross section; and
a plurality of batteries disposed within the diamond shaped cross section.

7. The apparatus of Claim 1 further comprising an over current fuse operational to reduce the potential overheating of the batteries.

8. The apparatus of Claim 1 further comprising:
an on-off switch assembly having at least one portion extending from the handle; and
the on-off switch assembly operable to activate the motor to rotate the drive shaft.

9. The apparatus of Claim 1 further comprising a trigger switch (262) operable to turn the motor on and off.

10. The apparatus of Claim 1 further comprising a timing circuit which may automatically turn off the motor after a set period of time to prevent draining the batteries.

11. The apparatus of Claim 1 further comprising:
an indicator (280) disposed on the handle proximate a distal end of the housing; and
the indicator operable to display the amount of power available in the power supply.

12. The apparatus of Claim 1 further comprising an over current fuse operational to reduce the potential overheating of the power supply.

## Patentansprüche

1. Eine Vorrichtung, die ein intraossäres Gerät (160) in einen Knochen und dazugehöriges Knochenmark einführen kann und Folgendes aufweist:
ein Gehäuse (232) mit einem Griff (236), der davon abgeht, und einer Antriebswelle (250), die aus einer Öffnung im Gehäuse verläuft, die Antriebswelle ist dabei bedienbar, um das intraossäre Gerät abnehmbar anzuschließen, der Griff ist als länglicher, hohler Behälter gestaltet, der so ausgelegt ist, dass er eine Stromversorgungseinheit (234) aufnehmen kann;
einen Motor (244), der innerhalb eines ersten Segments (232a) des Gehäuses angebracht und drehbar mit der Antriebswelle verbunden ist;
eine Stromversorgungseinheit (234) und ein dazugehöriger Stromkreis (274), die den Motor mit Strom versorgen können, die Stromversorgungseinheit ist dabei im Griff (236) untergebracht;
eine Getriebeanordnung (246), die zwischen und drehbar mit dem Motor und der Antriebswelle verbunden ist, wobei die Getriebeanordnung in einem zweiten Segment (232b) des Gehäuses untergebracht ist;
der Motor und die Getriebeanordnung haben dabei entsprechende im Allgemeinen zylindrische Konfigurationen;
die Getriebeanordnung mit einem nominalen Außendurchmesser-Teil, der kleiner ist als ein entsprechender nominaler Außendurchmesser-Teil des Motors;
wobei die Rotationsachse der Antriebswelle an der Rotationsachse des Motors ausgerichtet ist; **dadurch gekennzeichnet, dass**
das erste Segment (232a) des Gehäuses einen größeren nominalen Außendurchmesser hat als das zweite Segment (232b) des Gehäuses.

2. Die Vorrichtung gemäß Anspruch 1, wobei das intraossäre Gerät darüberhinaus Folgendes aufweist:
eine Durchstoßanordnung, die teilweise durch eine im Allgemeinen hohle, längliche Kanüle (210) definiert ist; und einen abnehmbaren, inneren Trokar (220), der verschiebbar in die Kanüle eingesetzt ist.

3. Die Vorrichtung gemäß Anspruch 1, die darüberhinaus Folgendes aufweist:
eine Auslöseanordnung (262), bei der mindestens ein Teil aus dem Gehäuse herausragt; die Auslöseanordnung ist dabei in der Lage, den Motor zu aktivieren, um die Antriebswelle zu drehen.

4. Die Vorrichtung gemäß Anspruch 1, wobei die Stromversorgungseinheit darüberhinaus vier Batterien (238) aufweist, die nebeneinander in einem im Allgemeinen diamantförmigen Muster angeordnet sind.

5. Die Vorrichtung gemäß Anspruch 1, wobei die Stromversorgungseinheit darüberhinaus Folgendes aufweist:
vier Batterien, die nebeneinander in einem im Allgemeinen diamantförmigen Muster angeordnet sind; und
eine fünfte Batterie, die an einem Ende der vier Batterien untergebracht ist.

6. Die Vorrichtung gemäß Anspruch 1, wobei die Stromversorgungseinheit Folgendes aufweist:
einen im Allgemeinen diamantförmigen Querschnitt; und
eine Vielzahl von Batterien, die in diesem diamantförmigen Querschnitt untergebracht sind.

7. Die Vorrichtung gemäß Anspruch 1, die darüberhinaus eine Überstromsicherung aufweist, die die potenzielle Überhitzung der Batterien verringern kann.

8. Die Vorrichtung gemäß Anspruch 1, die darüberhinaus Folgendes aufweist:
eine On-Off-Schalteranordnung, wobei mindestens ein Teil aus dem Griff herausragt;
und die On-Off-Schalteranordnung dabei in der Lage ist, den Motor zu aktivieren, um die Antriebswelle zu drehen.

9. Die Vorrichtung gemäß Anspruch 1, die darüberhinaus einen Auslöseschalter (262) aufweist, der den Motor ein- und ausschalten kann.

10. Die Vorrichtung gemäß Anspruch 1, die darüberhinaus einen Zeitgeber aufweist, der den Motor nach einer voreingestellten Zeitdauer automatisch ausschalten kann, um zu verhindern, dass die Batterien entleert werden.

11. Die Vorrichtung gemäß Anspruch 1, die darüberhinaus Folgendes aufweist:
eine Anzeige (280), die am Griff in der Nähe des proximalen Endes des Gehäuses angebracht ist; und
die Anzeige zeigt dabei die Strommenge an, die in der Stromversorgungseinheit noch verfügbar ist.

12. Die Vorrichtung gemäß Anspruch 1, die darüberhinaus eine Überstromsicherung aufweist, die die potenzielle Überhitzung der Stromversorgungseinheit verringern kann.

## Revendications

1. Un appareil conçu de façon à insérer un dispositif intraosseux (160) dans un os et une moelle osseuse associée comprenant :
un logement (232) avec une poignée (236) s'étendant à partir de celui-ci et un arbre d'entraînement (250) s'étendant à partir d'une ouverture dans le logement, l'arbre d'entraînement étant conçu de façon à entrer en prise de manière libérable avec le dispositif intraosseux, la poignée étant configurée sous la forme d'un réceptacle allongé et creux dimensionné de façon à recevoir une alimentation électrique (234),
un moteur (244) disposé à l'intérieur d'un premier segment (232a) du logement et en prise rotative avec l'arbre d'entraînement,
une alimentation électrique (234) et un circuit électrique associé (274) conçu de façon à alimenter le moteur, l'alimentation électrique étant disposée à l'intérieur de la poignée (236),
un ensemble à engrenages (246) disposé entre et en prise rotative avec le moteur et l'arbre d'entraînement, l'ensemble à engrenages étant disposé à l'intérieur d'un deuxième segment (232b) du logement,
le moteur et l'ensemble à engrenages possédant des configurations généralement cylindriques respectives,
l'ensemble à engrenages possédant une partie diamètre extérieur nominale plus petite qu'une partie diamètre extérieur nominale correspondante du moteur,
où l'axe de rotation de l'arbre d'entraînement est aligné avec l'axe de rotation du moteur, **caractérisé en ce que**
le premier segment (232a) du logement possède un diamètre extérieur nominal plus grand que le deuxième segment (232b) du logement.

2. L'appareil selon la Revendication 1 où le dispositif intraosseux comprend en outre :
un ensemble pénétrateur défini en partie par une canule allongée généralement creuse, (210) et
un trocart intérieur amovible (220) disposé de manière coulissable à l'intérieur de la canule.

3. L'appareil selon la Revendication 1 comprenant en outre :
un ensemble déclencheur (262) possédant au moins une partie s'étendant à partir du logement, et
l'ensemble déclencheur étant conçu de façon à activer le moteur de façon à mettre en rotation l'arbre d'entraînement.

4. L'appareil selon la Revendication 1 où l'alimentation électrique comprend en outre quatre batteries (238) disposées adjacentes l'une à l'autre selon un motif généralement en forme de losange.

5. L'appareil selon la Revendication 1 où l'alimentation électrique comprend en outre :
quatre batteries disposées adjacentes l'une à l'autre selon un motif généralement en forme de losange, et
une cinquième batterie disposée à une extrémité des quatre batteries.

6. L'appareil selon la Revendication 1 où l'alimentation électrique comprend :
une section transversale généralement en forme de losange, et
une pluralité de batteries disposées à l'intérieur de la section transversale en forme de losange.

7. L'appareil selon la Revendication 1 comprenant en outre un fusible de surintensité conçu de façon à réduire la surchauffe potentielle des batteries.

8. L'appareil selon la Revendication 1 comprenant en outre :
un ensemble commutateur marche-arrêt possédant au moins une partie s'étendant à partir de la poignée, et
l'ensemble commutateur marche-arrêt étant conçu de façon à activer le moteur de façon à mettre en rotation l'arbre d'entraînement.

9. L'appareil selon la Revendication 1 comprenant en outre un commutateur déclencheur (262) conçu de façon à démarrer et arrêter le moteur.

10. L'appareil selon la Revendication 1 comprenant en outre un circuit de synchronisation qui peut automatiquement arrêter le moteur après une période de temps définie de façon à prévenir un épuisement des batteries.

11. L'appareil selon la Revendication 1 comprenant en outre :
un indicateur (280) disposé sur la poignée à proximité d'une extrémité distale du logement, et
l'indicateur étant conçu de façon à afficher la quantité d'énergie électrique disponible dans l'alimentation électrique.

12. L'appareil selon la Revendication 1 comprenant en outre un fusible de surintensité conçu de façon à réduire la surchauffe potentielle de l'alimentation électrique.
